(19) Europäisches Patentamt
European Patent Office
Office européen des brevets

(11) Veröffentlichungsnummer: **0 540 823 A1**

(12) # EUROPÄISCHE PATENTANMELDUNG

(21) Anmeldenummer: **92111641.4**

(22) Anmeldetag: **09.07.92**

(51) Int. Cl.5: **C07C 309/17**, C11D 1/06, C11D 1/12, C11D 3/20

(30) Priorität: **07.11.91 DE 4136579**

(43) Veröffentlichungstag der Anmeldung: **12.05.93 Patentblatt 93/19**

(84) Benannte Vertragsstaaten: **BE DE DK ES FR GB IT NL PT**

(71) Anmelder: **REWO Chemische Werke GmbH
Industriegebiet West
W-6497 Steinau an der Strasse(DE)**

(72) Erfinder: **Hamann, Ingo, Dr. Dipl.-Chem.
Salmünsterer Strasse 13
W-6482 Bad Orb(DE)**
Erfinder: **Hohn, Elke
Struthrain 20
W-6490 Schlüchtern(DE)**
Erfinder: **Köhle, Hans-Jürgen, Dr. Dipl.-Chem.
Lotichiusstrasse 13
W-6490 Schlüchtern(DE)**

(54) **Polyolpolyethersulfosuccinate, Verfahren zu ihrer Herstellung und ihre Verwendung.**

(57) Die Erfindung betrifft Fettsäuremonoglyceridpolyglykolethersulfosuccinate der allgemeinen Formel (I)

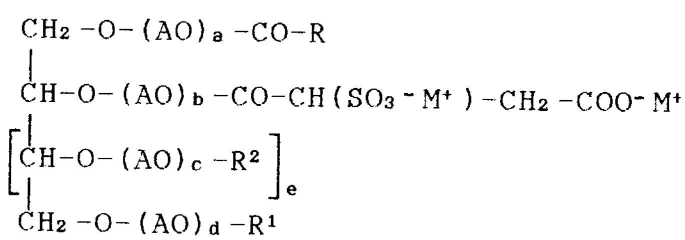

worin

| | |
|---|---|
| R = | ein geradkettiger oder verzweigter Alkyl- oder Alkylenrest mit 7 - 21 C-Atomen, |
| $R^1$ = | H oder -CO-R, |
| $R^2$ = | unabhängig voneinander H oder der Rest $-CO-CH(SO_3^-M^+)-CH_2-COO^-M^+$ sein kann, |
| A = | gleich oder verschiedene Alkylenreste mit 2 - 3 C-Atomen sind, |
| a,b,c,d = | gleich oder verschieden 0 - 5 sein können, wobei a + b + c • e + d = 2 - 25, |
| e = | 1 - 3, |
| $M^+$ = | Alkalimetallkation, Ammoniumion bedeuten, |

ihre Herstellung und ihre Verwendung.

EP 0 540 823 A1

Gegenstand der Erfindung sind neue Polyolpolyethersulfosuccinate, Verfahren zu ihrer Herstellung und ihre Verwendung als Reinigungsmittel und für kosmetische Präparate.

Kosmetische Präparate, insbesondere solche, die in den Bereich der Haar- und Körperreinigung fallen, wie Duschbäder, Schaumbäder, Haarshampoos, flüssige Seifen, enthalten als Reinigungskomponenten hauptsächlich Aniontenside wie Carboxylate, Alkylsulfate und Alkylethersulfate, Sulfosuccinate.

Diese Zubereitungen sollen die Hautoberfläche reinigen, vorzugsweise nur den auf ihr anhaftenden Film, welcher aus Körperausscheidungen wie Schweiß und Fetten, Hautschuppen oder abgelagertem Schmutz aus der Umgebung bestehen kann. Die Reinigungsmittel sollen keinesfalls die Haut auslaugen, sie reizen oder ihre natürliche Funktion beeinträchtigen.

Da die Präparate aber bei ihrer häufigen – in den letzten Jahren fast täglichen – Anwendung zu Irritationen der Haut führen können, werden zur Verbesserung der Haut- und Augenschleimhautverträglichkeit häufig sogenannte milde Tenside mitverwendet, wie beispielsweise Betaine, Proteinderivate, Ampholyte, Alkylethercarboxylate und Sulfosuccinate.

Insbesondere für Babypflegemittel und Babyshampoos wird besonderer Wert auf extrem niedrige Gehalte an die Haut und Augenschleimhaut reizende Substanzen gelegt. Die konventionell wegen ihrer ausgezeichneten Reinigungs- und Schaumbildungseigenschaften verwendeten anionischen Tenside können durch die bekannten milden Co-Tenside in ihrer Irritationswirkung zwar weitgehend gemildert werden, in der Praxis werden aber noch Verbesserungen insbesondere in Richtung auf Augenschleimhautverträglichkeit gefordert.

In hohen Konzentrationen oder allein sind die milden Tenside zwar weitgehend irritationsfrei, zeigen dann jedoch keine praxisgerechten Schäumungs- und Reinigungseigenschaften und unbefriedigende Viskositäten.

Ein weiteres Kriterium für die Brauchbarkeit der oberflächenaktiven Substanzen ist ihre Toxizität. Die Toxizität liegt in den Tensiden selbst oder ihren durch Wechselwirkung mit den Bestandteilen der Rezeptur entstehenden Produkten.

So werden die wegen ihrer vorteilhaften anwendungstechnischen und hautpflegenden Eigenschaften bislang gern mitverwendeten Fettsäurealkanolamide in letzter Zeit nur noch ungern in kosmetischen Präparaten eingesetzt, da der bei der Herstellung verbleibende Restgehalt an freiem Diethanolamin nicht ausgeschlossen werden und damit Anlaß zur Nitrosaminbildung sein kann (EP-A-0 306 843). N-Nitrosamine wirken im Tierversuch krebserzeugend.

Aufgrund des gewachsenen Umweltbewußtseins werden weiterhin Produkte verlangt, welche auf natürlichen nachwachsenden Rohstoffen basieren, keinerlei Toxizität aufweisen und in den kommunalen Kläranlagen schnell und vollständig ohne toxische Zwischenprodukte abgebaut werden können.

Die in der Praxis eingeführten Produkte auf Basis von mehrwertigen Alkoholen wie beispielsweise die Mono- und/oder Difettsäureesteralkoxylate auf Basis von Glycerin weisen zwar niedrige Toxizitäten auf und sind milde, die Haut nicht reizende Produkte, welche hinsichtlich ihrer Reinigungswirkung aber nicht den Erfordernissen der Praxis entsprechen.

Aufgabe der vorliegenden Erfindung ist es, diese Nachteile des Standes der Technik zu überwinden und milde, hautfreundliche Reinigungsmittel für Haushalt und Industrie und kosmetische Präparate, insbesondere solche, die in den Bereich der Haar- und Körperreinigung fallen, zur Verfügung zu stellen.

Diese Aufgabe wird gelöst durch die erfindungsgemäß mitverwendeten Sulfosuccinate auf Basis von alkoxylierten Estergruppen enthaltenden Polyolen mit >3 OH-Gruppen/Mol. Gegenstand der Erfindung sind Polyolpolyethersulfosuccinate der allgemeinen Formel (I)

$$\begin{array}{l} CH_2-O-(AO)_a-CO-R \\ | \\ CH-O-(AO)_b-CO-CH(SO_3^-M^+)-CH_2-COO^-M^+ \\ | \\ \left[ CH-O-(AO)_c-R^2 \right]_e \\ | \\ CH_2-O-(AO)_d-R^1 \end{array} \qquad (I)$$

worin

R = ein geradkettiger oder verzweigter Alkyl- oder Alkylenrest mit 7 - 21 C-Atomen,

$R^1$ = H oder -CO-R,

$R^2$ = unabhängig voneinander H oder der Rest $-CO-CH(SO_3^-M^+)-CH_2-COO^-M^+$ sein kann,

A = gleich oder verschiedene Alkylenreste mit 2 − 3 C−Atomen sind,
a,b,c,d = gleich oder verschieden 0 − 5 sein können, wobei

$$a + b + c \cdot e + d = 2 - 25,$$

e = 1 − 3,
$M^+$ = Alkalimetallkation, Ammoniumion bedeuten.

Ein weiterer Gegenstand der Erfindung ist ein Verfahren zur Herstellung von Verbindungen der allgemeinen Formel (I).

Weitere Gegenstände der Erfindung sind wässrige Haarreinigungs− und Pflegemittel, Spülmittel, Hautpflege−und reinigungsmittel, welche dadurch gekennzeichnet sind, daß sie 1 − 10 Gew.−Teile mindestens einer der Verbindungen der allgemeinen Formel (I), definierte Mengen mindestens eines Tensids aus der Gruppe der nichtionischen, amphoteren, zwitterionischen und ionischen Tenside, vorzugsweise 0,1 bis 30 Gew.−Teile, gegebenenfalls 0,1 bis 15 Gew.−Teile einer der üblichen Mittel aus der Gruppe der Zusatz− und Hilfsstoffe, Verdickungsmittel, Duftstoffe, Konservierungsmittel, Farbstoffe, Pflanzenextrakte und gegebenenfalls ad 100 Wasser enthalten.

Weitere Gegenstände der Erfindung sind durch die Ansprüche gekennzeichnet.

Als Alkohole für die Herstellung der erfindungsgemäßen Verbindungen werden Polyolverbindungen mit mehr als drei Hydroxylgruppen eingesetzt. Vorzugsweise sind dies handelsübliche Zuckeralkohole wie Erythritol, Xylitol und insbesondere die 6−wertigen Reduktionsprodukte der Monosaccharide wie Mannitol und Sorbitol. Je nach Anforderungen an das Endprodukt kann hier hochgereinigte Qualität oder die handelsübliche Ware von technischer Reinheit eingesetzt werden.

Im allgemeinen wird erfindungsgemäß das Verfahren so geführt, daß in erster Stufe die Polyolalkoxylate durch Anlagerung von Alkylenoxiden wie Ethylenoxid, Propylenoxid in Gegenwart eines basischen Katalysators bei Temperaturen von 120 − 180 °C an die Polyole hergestellt werden, daß in der zweiten Stufe durch Ver− bzw. Umesterung mit Fettsäuren bzw. Fettsäureestern bei 120 − 180 °C, gegebenenfalls in Gegenwart eines Katalysators, die Polyolalkoxylatfettsäureester und in dritter Stufe durch Addition von Maleinsäureanhydrid bei 60 − 80 °C der Halbester hergestellt und dieser in letzter Stufe bei 60 − 80 °C mit wässriger Natriumsulfitlösung zu dem Salz der entsprechenden Sulfonsäure umgesetzt wird (J. Am. Oil Chem. Soc., 38, 517 − 520 (1961), US−PS 3 640 998).

Die erfindungsgemäßen Verbindungen haben Alkoxylierungsgrade mit a, b, c und d von jeweils 0 − 5, vorzugsweise 1 − 3, wobei a+b+c+d = 2 − 25, vorzugsweise 5 − 20, sein kann. Diese Werte sind als statistische Mittelwerte zu betrachten.

Als Fettsäuren werden die einbasischen Säuren oder deren Alkylester mit 8 − 22, vorzugsweise 12 − 18, C−Atomen − bevorzugt die natürlich vorkommenden − verwendet wie Laurinsäure, Palmitinsäure, Stearinsäure, Ölsäure, Linolsäure, Linolensäure, Ricinolsäure, Kokosfettsäure oder deren Mischungen (vgl. J. Am. Oil Chem. Soc. 38, 517 − 520 (1961) und EP−A−0 190 779).

Die Reaktion zum Sulfosuccinat erfolgt mit Maleinsäureanhydrid und anschließender Sulfierung mit Natriumsulfit nach an sich bekannten Verfahren (DE−OS 2 700 072).

Dabei wird pro Mol umzusetzende Hydroxylgruppe mit einem Mol Maleinsäureanhydrid bei 60 − 80 °C bis zur vollständigen Reaktion des Anhydrids gerührt. Der Maleinsäurehalbester wird anschließend in eine wässrige Natriumsulfit−Lösung gegeben (1 Equivalent Sulfit pro Equivalent Halbester) und bei 60 − 80 °C sulfiert, bis das Sulfit vollständig abreagiert ist. Dann wird das wässrige Produkt pH−neutral eingestellt.

Die erfindungsgemäßen Verbindungen sind durch die allgemeine Formel (I) in idealisierter Form dargestellt. Die technischen Mischungen enthalten neben den gewünschten Estern noch geringe Anteile von Verbindungen mit anderem Veresterungsgrad und auch die angegebenen Alkoxylierungsgrade sind statistische Mittelwerte.

Für die erfindungsgemäße Verwendung ist eine Reinigung nicht erforderlich, das heißt, die technischen Mischungen sind als solche direkt weiter verwendbar.

Die erfindungsgemäßen Mischungen für Reinigungszwecke und Kosmetik liegen im allgemeinen als wässrige Mittel oder als wässrige alkoholische Lösungen, Cremes, Emulsionen, Gele vor und können zur Anpassung an den vorgesehenen Anwendungszweck noch die jeweils üblichen Hilfs− und Zusatzstoffe enthalten, welche für die Herstellung von Reinigungsmitteln und kosmetischen Präparaten im Bereich der Haar− und Körperreinigung, das heißt für Duschbäder, Schaumbäder, Shampoos, flüssige Seifen, Babypflege− und waschmittel sowie für milde Reinigungsmittel wie Geschirrspülmittel für manuelle Anwendung, Neutralreiniger und Allzweckreiniger übilch sind, mitverwendet werden.

Für die kosmetischen Anwendungen können die auf diesen Gebieten üblichen Tenside, Duftstoffe, Konservierungsmittel, Farbstoffe, Pflanzenextrakte bzw. sonstige kosmetische Additive mitverwendet werden.

Als Tenside kommen neben den bekannten Betainen, amphoteren und nichtionischen Verbindungen für die reinigenden Rezepturen insbesondere die anionischen Tenside wie Carboxylate, Alkylsulfate, Alkylethersulfate, Alkylsulfonate, Alkylethersulfonate, Alkylsulfosuccinate in Betracht. Erfindungsgemäß bevorzugt werden Alkylamidobetaine wie REWOTERIC$^R$ [1)] AM B 13 und AM B 14, Carboxyglycinate wie REWOTERIC$^R$ AM 2 C NM, Carboxypropionate wie REWOTERIC$^R$ AM KSF 40, Sulfobetaine wie REWOTERIC$^R$ AM CAS, anionische Tenside wie Ethersulfate REWOPOL$^R$ [2)] NL 3, Ethercarboxylate wie REWOPOL$^R$ CLN 100, Sulfosuccinate wie REWOPOL$^R$ SB FA 30, REWOPOL$^R$ SBZ, REWODERM$^R$ [3)] SPS, nichtionische Tenside wie Glycerinfettsäureesterethoxylate wie REWODERM$^R$ ES 90, Glycerinmonostearat wie REWOMUL$^R$ [4)] MG, Cetylalkohol ([1)], [2)], [3)], [4)] = Warenzeichen der Fa. REWO Chemische Werke GmbH, Steinau an der Straße).

Neben den erfindungsgemäßen Verbindungen, welche in Mengen von 1 – 10 Gew.-Teilen, insbesondere 2 – 8 Gew.-Teilen, bei Shampoos und 1 – 7 Gew.-Teilen, vorzugsweise 1 – 5 Gew.-Teilen, bei Cremes eingesetzt werden, werden die anderen Tenside bei den Shampoos üblicherweise in Mengen von 1 – 20 Gew.-Teilen, vorzugsweise 1 – 15 Gew.-Teilen, bei den Cremes in Mengen von 1 – 10 Gew.-Teilen, vorzugsweise 2 – 5 Gew.-Teilen, mitverwendet.

Als Verdickungsmittel werden 1 – 8 Gew.-Teile der auf diesem Gebiet üblichen Verbindungen wie Glycerinfettsäureesterethoxylate, Fettalkoholethoxylate, Fettsäurealkylolamide und die üblichen Alkali-, Erdalkali- und Ammoniumsalze, welche bei 20 °C in Mengen von mindestens 1 Gew.-% in Wasser löslich sind, wie insbesondere NaCl, $NH_4Cl$ eingesetzt.

## Analysenmethoden

### Feststoffgehalt:

Der Gehalt wird bestimmt durch Erhitzen und Trocknen der Substanz bis zur Gewichtskonstanz bei 105 °C.

### Verseifungszahl (VZ):

Die Verseifungszahl ist ein Maß für die in Fetten und technischen Fettsäuren enthaltenen freien und gebundenen Säuren. Sie gibt die Anzahl Milligramm Kaliumhydroxid an, die notwendig ist, um 1 Gramm Substanz oder technische Fettsäuren zu verseifen (mg KOH/g). Diese Werte werden bestimmt nach den Einheitsmethoden der Deutschen Gesellschaft für Fettchemie (DGF): DGF C-V3.

### Hydroxylzahl (OHZ):

Die Hydroxylzahl ist ein Maß für die in einer Substanz enthaltenen Hydroxylgruppen. Sie gibt die Anzahl Milligramm Kaliumhydroxid an, die notwendig ist, um die von 1 Gramm Substanz bei der Acetylierung verbrauchte Essigsäure zu neutralisieren (mg KOH/g). Diese Werte werden bestimmt nach den Einheitsmethoden der Deutschen Gesellschaft für Fettchemie (DGF): DGF C-V17a.

### Benzavlon-WAS:

Der in den nachfolgenden Beispielen angegebene Gehalt an waschaktiver Substanz (B-WAS) wird durch die übliche Zweiphasentitration mit Benzalkoniumchlorid gegen Methylenblau als Indikator titriert (vgl. S. R. Epton, Nature (London), 160, 795 (1967)).

## A) Synthesebeispiele

### Beispiel 1

In einem Laborautoklaven werden 848 g (4,66 Mol) Sorbitol vorgelegt und bei 100 °C geschmolzen. Bei einer Temperatur von 130 – 150 °C werden nach Zusatz von 1 g Natriumhydroxid 410 g (9,32 Mol) Ethylenoxid eingeleitet, so daß der Druck im Reaktionsgefäß nicht über 4 bar ansteigt. Nach Beendigung der Umsetzung weist das Produkt eine Hydroxylzahl von 1174 auf.

In einem 1 Liter-4-Halskolben mit Rührer, Innenthermometer, Inertgaszuleitung und Destillationsbrücke werden 270 g (1 Mol) Sorbitol-Ethoxylat mit 0,4 g Tetraisopropyltitanat versetzt und nach langsamer Zugabe von 235 g (1 Mol) Kokosfettsäuremethylester $C_{12}$ - $C_{18}$ unter einer Stickstoffatmoshäre auf 130 °C erwärmt. Das entstehende Methanol wird unter Normaldruck kontinuierlich abdestilliert; gegebenenfalls wird die Reaktion durch Anlegen von Vakuum vervollständigt. Das Umesterungsprodukt besitzt eine Hydroxylzahl von 575 und eine Verseifungszahl von 114.

260 g (0,55 Mol) Sorbitolmonoester werden in einem 500 ml 4-Halskolben mit Rührer, Innenthermometer, Inertgaszuleitung und Rückflußkühler mit 108 g (1,1 Mol) Maleinsäureanhydrid versetzt und unter einer Stickstoffatmosphäre 2 h auf 80 - 90 °C erwärmt. 280 g des Maleinsaurehalbesters werden anschließend in eine auf 70 °C erwärmte Lösung von 111 g Natriumsulfit in 580 ml Wasser gegossen und bei dieser Temperatur gerührt, bis der $SO_2$-Gehalt der Lösung <0,01 % beträgt. Es entsteht eine Lösung mit einem Feststoffgehalt von 40,0 % und einer Benzavlon-WAS von 25,8 %.

Beispiel 2

In einem Laborautoklaven werden 414 g (2,27 Mol) Sorbitol vorgelegt und bei 100 °C geschmolzen. Bei einer Temperatur von 130 - 150 °C werden nach Zusatz von 1 g Natriumhydroxid 300 g (6,82 Mol) Ethylenoxid eingeleitet, so daß der Druck im Reaktionsgefäß nicht über 4 bar ansteigt. Nach Beendigung der Umsetzung weist das Produkt eine Hydroxylzahl von 1065 auf.

In einem 2 Liter-4-Halskolben mit Rührer, Innenthermometer, Inertgaszuleitung und Destillationsbrücke werden 585 g (1,86 Mol) Sorbitol-Ethoxylat mit 2,9 g Kaliumcarbonat versetzt und nach langsamer Zugabe von 235 g (1,86 Mol) Kokosfettsäuremethylester $C_{12}$ - $C_{18}$ unter einer Stickstoffatmosphäre auf 125 °C erwärmt. Das entstehende Methanol wird unter Normaldruck kontinuierlich abdestilliert; gegebenenfalls wird die Reaktion durch Anlegen von Vakuum vervollständigt. Das Umesterungsprodukt besitzt eine Hydroxylzahl von 634 und eine Verseifungszahl von 128.

207 g (0,4 Mol) Sorbitolmonoester werden in einem 500 ml 4-Halskolben mit Rührer, Innenthermometer, Inertgaszuleitung und Rückflußkühler mit 118 g (1,2 Mol) Maleinsäureanhydrid versetzt und unter einer Stickstoffatmosphäre 2 h auf 80 - 90 °C erwärmt.

280 g des Maleinsäurehalbesters werden anschließend in eine auf 70 °C erwärmte Lösung von 137 g Natriumsulfit in 625 ml Wasser gegossen und bei dieser Temperatur gerührt, bis der $SO_2$-Gehalt der Lösung <0,01 % beträgt. Es entsteht eine Lösung mit einem Feststoffgehalt von 43,7 % und einer Benzavlon-WAS von 40,6 %.

Beispiel 3

259 g (0,5 Mol) Sorbitolmonoester aus Beispiel 2 werden in einem 500 ml 4-Halskolben mit Rührer, Innenthermometer, Inertgaszuleitung und Rückflußkühler mit 98 g (1 Mol) Maleinsäureanhydrid versetzt und unter einer Stickstoffatmosphäre 2 h auf 80 - 90 °C erwärmt.

300 g des Maleinsäurehalbesters werden anschließend in eine auf 70 °C erwärmte Lösung von 111 g Natriumsulfit in 615 ml Wasser gegossen und bei dieser Temperatur gerührt, bis der $SO_2$-Gehalt der Lösung <0,01 % beträgt. Es entsteht eine Lösung mit einem Feststoffgehalt von 40,4 % und einer Benzavlon-WAS von 28,5 %.

Beispiel 4

In einem Laborautoklaven werden 520 g (2,85 Mol) Sorbitol vorgelegt und bei 100 °C geschmolzen. Bei einer Temperatur von 130 - 150 °C werden nach Zusatz von 1 g Natriumhydroxid 630 g (14,3 Mol) Ethylenoxid eingeleitet, so daß der Druck im Reaktionsgefäß nicht über 4 bar ansteigt. Nach Beendigung der Umsetzung weist das Produkt eine Hydroxylzahl von 818 auf.

In einem 1 Liter-4-Halskolben mit Rührer, Innenthermometer, Inertgaszuleitung und Destillationsbrücke werden 402 g (1 Mol) Sorbitol-Ethoxylat mit 1,2 g Methansulfonsäure versetzt und nach Zugabe von 220 g (1 Mol) Kokosfettsäure $C_{12}$ - $C_{10}$ unter einer Stickstoffatmosphäre auf 180 °C erwärmt. Das entstehende Reaktionswasser wird unter Normaldruck kontinuierlich abdestilliert; gegebenenfalls wird die Reaktion durch Anlegen von Vakuum vervollständigt. Das Veresterungsprodukt besitzt eine Hydroxylzahl von 404 und eine Verseifungszahl von 96.

200 g (0,33 Mol) Sorbitolmonoester werden in einem 500 ml 4-Halskolben mit Rührer, Innenthermometer, Inertgaszuleitung und Rückflußkühler mit 65 g (0,66 Mol) Maleinsäureanhydrid versetzt und unter einer Stickstoffatmosphäre 2 h auf 80 - 90 °C erwärmt. 230 g des Maleinsäurehalbesters werden

anschließend in eine auf 70 °C erwärmte Lösung von 76 g Natriumsulfit in 460 ml Wasser gegossen und bei dieser Temperatur gerührt, bis der $SO_2$-Gehalt der Lösung <0,01 % beträgt. Es entsteht eine klare helle Lösung mit einem Feststoffgehalt von 40,0 % und einer Benzavlon-WAS von 30,4 %.

Beispiel 5

In einem Laborautoklaven werden 457 g (2,51 Mol) Sorbitol vorgelegt und bei 100 °C geschmolzen. Bei einer Temperatur von 130 - 150 °C werden nach Zusatz von 1 g Natriumhydroxid 728 g (12,6 Mol) Propylenoxid eingeleitet, so daß der Druck im Reaktionsgefäß nicht über 4 bar ansteigt. Nach Beendigung der Umsetzung weist das Produkt eine Hydroxylzahl von 705 auf.

In einem 1 Liter-4-Halskolben mit Rührer, Innenthermometer, Inertgaszuleitung und Destillationsbrücke werden 472 g (1 Mol) Sorbitol-Propoxylat mit 2,5 g Kaliumcarbonat versetzt und nach Zugabe von 235 g (1 Mol) Kokosfettsäuremethylester $C_{12}$ - $C_{18}$ unter einer Stickstoffatmosphäre auf 180 °C erwärmt. Das entstehende Methanol wird unter Normaldruck kontinuierlich abdestilliert; gegebenenfalls wird die Reaktion durch Anlegen von Vakuum vervollständigt. Das Umesterungsprodukt besitzt eine Hydroxylzahl von 380 und eine Verseifungszahl von 78.

270 g (0,4 Mol) Sorbitolmonoester werden in einem 500 ml 4-Halskolben mit Rührer, Innenthermometer, Inertgaszuleitung und Rückflußkühler mit 118 g (1,2 Mol) Maleinsäureanhydrid versetzt und unter einer Stickstoffatmosphäre 2 h auf 80 - 90 °C erwärmt. 340 g des Maleinsäurehalbesters werden anschließend in eine auf 70 °C erwärmte Lösung von 140 g Natriumsulfit in 720 ml Wasser gegossen und bei dieser Temperatur gerührt, bis der $SO_2$-Gehalt der Lösung <0,01 % beträgt. Es entsteht eine klare helle Lösung mit einem Feststoffgehalt von 40,1 % und einer Benzavlon-WAS von 19,2 %.

Beispiel 6

In einem Laborautoklaven werden 394 g (2,16 Mol) Sorbitol vorgelegt und bei 100 °C geschmolzen. Bei einer Temperatur von 130 - 150 °C werden nach Zusatz von 1 g Natriumhydroxid 950 g (21,6 Mol) Ethylenoxid eingeleitet, so daß der Druck im Reaktionsgefäß nicht über 4 bar ansteigt. Nach Beendigung der Umsetzung weist das Produkt eine Hydroxylzahl von 518 auf.

In einem 1 Liter-4-Halskolben mit Rührer, Innenthermometer, Inertgaszuleitung und Destillationsbrücke werden 373 g (0,6 Mol) Sorbiteo-Ethoxylat mit 2 g Kaliumcarbonat versetzt und nach langsamer Zugabe von 141 g (0,6 Mol) Kokosfettsäuremethylester $C_{12}$ - $C_{18}$ unter einer Stickstoffatmosphäre auf 130 °C erwärmt. Das entstehende Methanol wird unter Normaldruck kontinuierlich abdestilliert; gegebenenfalls wird die Reaktion durch Anlegen von Vakuum vervollständigt. Das Umesterungsprodukt besitzt eine Hydroxylzahl von 315 und eine Verseifungszahl von 61.

206 g (0,25 Mol) Sorbitolmonoester werden in einem 500 ml 4-Halskolben mit Rührer, Innenthermometer, Inertgaszuleitung und Rückflußkühler mit 49 g (0,5 Mol) Maleinsäureanhydrid versetzt unter einer Stickstoffatmosphäre 2 h auf 80 - 90 °C erwärmt.

204 g des Maleinsäurehalbesters werden anschließend in eine auf 70 °C erwärmte Lösung von 53 g Natriumsulfit in 390 ml Wasser gegossen und bei dieser Temperatur gerührt, bis der $SO_2$-Gehalt der Lösung <0,01 % beträgt. Es entsteht eine Lösung mit einem Feststoffgehalt von 40,2 % und einer Benzavlon-WAS von 33,1 %.

Beispiel 7

In einem 1 Liter-4-Halskolben mit Rührer, Innenthermometer, Inertgaszuleitung und Destillationsbrücke werden 689 g (1,11 Mol) Sorbitol-Ethoxylat aus Beispiel 6 mit 3,4 g Kaliumcarbonat versetzt und nach langsamer Zugabe von 390 g (1,66 Mol) Kokosfettsäuremethylester $C_{12}$ - $C_{18}$ unter einer Stickstoffatmosphäre auf 130 °C erwärmt. Das entstehende Methanol wird unter Normaldruck kontinuierlich abdestilliert; gegebenenfalls wird die Reaktion durch Anlegen von Vakuum vervollständigt. Das Umesterungsprodukt besitzt eine Hydroxylzahl von 271 und eine Verseifungszahl von 87.

278 g (0,3 Mol) Sorbitolmonoester werden in einem 500 ml 4-Halskolben mit Rührer, Innenthermometer, Inertgaszuleitung und Rückflußkühler mit 88 g (0,9 Mol) Maleinsäureanhydrid versetzt und unter einer Stickstoffatmosphäre 2 h auf 80 - 90 °C erwärmt. 330 g des Maleinsäurehalbesters werden anschließend in eine auf 70 °C erwärmte Lösung von 108 g Natriumsulfit in 660 ml Wasser gegossen und bei dieser Temperatur gerührt, bis der $SO_2$-Gehalt der Lösung <0,01 % beträgt. Es entsteht eine Lösung mit einem Feststoffgehalt von 39,0 % und einer Benzavlon-WAS von 34,3 %.

Beispiel 8

370 g (0,4 Mol) Sorbitolmonoester aus Beispiel 6 werden in einem 500 ml 4-Halskolben mit Rührer, Innenthermometer, Inertgaszuleitung und Rückflußkühler mit 39 g (0,4 Mol) Maleinsäureanhydrid versetzt und unter einer Stickstoffatmosphäre 2 h auf 80 - 90 °C erwärmt. 375 g des Maleinsäurehalbesters werden anschließend in eine auf 70 °C erwärmte Lösung von 49 g Natriumsulfit in 640 ml Wasser gegossen und bei dieser Temperatur gerührt, bis der $SO_2$-Gehalt der Lösung <0,01 % beträgt. Es entsteht eine Lösung mit einem Feststoffgehalt von 39,6 % und einer Benzavlon-WAS von 23,9 %.

Beispiel 9

In einem Laborautoklaven werden 394 g (2,16 Mol) Sorbitol vorgelegt und bei 100 °C geschmolzen. Bei einer Temperatur von 130 - 150 °C werden nach Zusatz von 1 g Natriumhydroxid 1900 g (43,2 Mol) Ethylenoxid eingeleitet, so daß der Druck im Reaktionsgefäß nicht über 4 bar ansteigt. Nach Beendigung der Umsetzung weist das Produkt eine Hydroxylzahl von 314 auf.

In einem 1 Liter-4-Halskolben mit Rührer, Innenthermometer, Inertgaszuleitung und Destillationsbrücke werden 425 g (0,4 Mol) Sorbitol-Ethoxylat mit 2 g Kaliumcarbonat versetzt und nach langsamer Zugabe von 94 g (0,4 Mol) Kokosfettsäuremethylester $C_{12}$ - $C_{18}$ unter einer Stickstoffatmosphäre auf 130 °C erwärmt. Das entstehende Methanol wird unter Normaldruck kontinuierlich abdestilliert; gegebenenfalls wird die Reaktion durch Anlegen von Vakuum vervollständigt. Das Umesterungsprodukt besitzt eine Hydroxylzahl von 207 und eine Verseifungszahl von 43.

253 g (0,2 Mol) Sorbitolmonoester werden in einem 500 ml 4-Halskolben mit Rührer, Innenthermometer, Inertgaszuleitung und Rückflußkühler mit 39 g (0,4 Mol) Maleinsäureanhydrid versetzt und unter einer Stickstoffatmosphäre 2 h auf 80 - 90 °C erwärmt. 249 g des Maleinsäurehalbesters werden anschließend in eine auf 70 °C erwärmte Lösung von 45 g Natriumsulfit in 440 ml Wasser gegossen und bei dieser Temperatur gerührt, bis der $SO_2$-Gehalt der Lösung <0,01 % beträgt. Es entsteht eine Lösung mit einem Feststoffgehalt von 40,0 % und einer Benzavlon-WAS von 36 9 %.

Beispiel 10

In einem 1 Liter-4-Halskolben mit Rührer, Innenthermometer, Inertgaszuleitung und Destillationsbrücke werden 242 g (0,23 Mol) Sorbitol-Ethoxylat aus Beispiel 9 mit 1,2 g Kaliumcarbonat versetzt und nach langsamer Zugabe von 107 g (0,46 Mol) Kokosfettsäuremethylester $C_{12}$ - $C_{18}$ unter einer Stickstof-fatmosphäre auf 130 °C erwärmt. Das entstehende Methanol wird unter Normaldruck kontinuierlich abdestilliert; gegebenenfalls wird die Reaktion durch Anlegen von Vakuum vervollständigt. Das Umesterungsprodukt besitzt eine Hydroxylzahl von 142 und eine Verseifungszahl von 73.

293 g (0,2 Mol) Sorbitolmonoester werden in einem 500 ml 4-Halskolben mit Rührer, Innenthermometer, Inertgaszuleitung und Rückflußkühler mit 39 g (0,4 Mol) Maleinsäureanhydrid versetzt und unter einer Stickstoffatmosphäre 2 h auf 80 - 90 °C erwärmt. 300 g des Maleinsäurehalbesters werden anschließend in eine auf 70 °C erwärmte Lösung von 48 g Natriumsulfit in 520 ml Wasser gegossen und bei dieser Temperatur gerührt, bis der $SO_2$-Gehalt der Lösung <0,01 % beträgt. Es entsteht eine Lösung mit einem Feststoffgehalt von 39,9 % und einer Benzavlon-WAS von 28,8 %.

Beispiel 11

In einem Laborautoklaven werden 523 g (3,44 Mol) Xylitol vorgelegt und bei 100 °C geschmolzen. Bei einer Temperatur von 150 - 180 °C werden nach Zusatz von 1 g Natriumhydroxid 757 g (17,2 Mol) Ethylenoxid eingeleitet, so daß der Druck im Reaktionsgefäß nicht über 4 bar ansteigt. Nach Beendigung der Umsetzung weist das Produkt eine Hydroxylzahl von 735 auf.

In einem 1 Liter-4-Halskolben mit Rührer, Innenthermometer, Inertgaszuleitung und Destillationsbrücke werden 372 g (1 Mol) Xylitol-Ethoxylat mit 1,8 g Kaliumcarbonat versetzt und nach langsamer Zugabe von 235 g (1 Mol) Kokosfettsäuremethylester $C_{12}$ - $C_{18}$ unter einer Stickstoffatmosphäre auf 130 °C erwärmt. Das entstehende Methanol wird unter Normaldruck kontinuierlich abdestilliert; gegebenenfalls wird die Reaktion durch Anlegen von Vakuum vervollständigt. Das Umesterungsprodukt besitzt eine Hydroxylzahl von 381 und eine Verseifungszahl von 95.

247 g (0,43 Mol) Xylitolmonoester werden in einem 500 ml 4-Halskolben mit Rührer, Innenthermometer, Inertgaszuleitung und Rückflußkühler mit 105 g (1,1 Mol) Maleinsäureanhydrid versetzt und unter einer Stickstoffatmosphäre 2 h auf 80 - 90 °C erwärmt. 300 g des Maleinsäurehalbesters werden anschließend

in eine auf 70 °C erwärmte Lösung von 121 g Natriumsulfit in 630 ml Wasser gegossen und bei dieser Temperatur gerührt, bis der $SO_2$-Gehalt der Lösung <0,01 % beträgt. Es entsteht eine Lösung mit einem Feststoffgehalt von 45,2 % und einer Benzavlon-WAS von 40,0 %.

Beispiel 12

In einem Laborautoklaven werden 261 g (1,72 Mol) Xylitol vorgelegt und bei 100 °C geschmolzen. Bei einer Temperatur von 150 - 180 °C werden nach Zusatz von 1 g Natriumhydroxid 757 g (17,2 Mol) Ethylenoxid eingeleitet, so daß der Druck im Reaktionsgefäß nicht über 4 bar ansteigt. Nach Beendigung der Umsetzung weist das Produkt eine Hydroxylzahl von 470 auf.

In einem 1 Liter-4-Halskolben mit Rührer, Innenthermometer, Inertgaszuleitung und Destillationsbrücke werden 414 g (0,7 Mol) Xylitol-Ethoxylat mit 2 g Kaliumcarbonat versetzt und nach langsamer Zugabe von 165 g (0,7 Mol) Kokosfettsäuremethylester $C_{12}$ - $C_{18}$ unter einer Stickstoffatmosphäre auf 130 °C erwärmt. Das entstehende Methanol wird unter Normaldruck kontinuierlich abdestilliert; gegebenenfalls wird die Reaktion durch Anlegen von Vakuum vervollständigt. Das Umesterungsprodukt besitzt eine Hydroxylzahl von 284 und eine Verseifungszahl von 68.

518 g (0,4 Mol) Xylitolmonoester werden in einem 1 Liter-4-Halskolben mit Rührer, Innenthermometer, Inertgaszuleitung und Rückflußkühler mit 78 g (0,8 Mol) Maleinsäureanhydrid versetzt und unter einer Stickstoffatmospähre 2 h auf 80 - 90 °C erwärmt. 350 g des Maleinsäurehalbesters werden anschließend in eine auf 70 °C erwärmte Lösung von 94 g Natriumsulfit in 660 ml Wasser gegossen und bei dieser Temperatur gerührt, bis der $SO_2$-Gehalt der Lösung <0,01 % beträgt. Es entsteht eine Lösung mit einem Feststoffgehalt von 39,8 % und einer Benzavlon-WAS von 34,6 %

Tabelle 1

| Beispiel | a+b+c+d | e | R | $R^1$ | $R^2$ | $R^2$ | $R^2$ |
|---|---|---|---|---|---|---|---|
| 1 | 2 EO | 3 | $C_{11}/C_{17}$ | H | H | H | H |
| 2 | 3 EO | 3 | $C_{11}/C_{17}$ | H | Sulfo-succinat | Sulfo-succinat | H |
| 3 | 3 EO | 3 | $C_{11}/C_{17}$ | H | Sulfo-succinat | H | H |
| 4 | 5 EO | 3 | $C_{11}/C_{17}$ | H | Sulfo-succinat | H | H |
| 5 | 5 PO | 3 | $C_{11}/C_{17}$ | H | Sulfo-succinat | Sulfo-succinat | H |
| 6 | 10 EO | 3 | $C_{11}/C_{17}$ | H | Sulfo-succinat | H | H |
| 7 | 10 EO | 3 | $C_{11}/C_{17}$ | $C_{11}/C_{17}$* | Sulfo-succinat | Sulfo-succinat | H |
| 8 | 10 EO | 3 | $C_{11}/C_{17}$ | $C_{11}/C_{17}$* | H | H | H |

Tabelle 1 (Fortsetzung)

| Beispiel | a+b+c+d | e | R | $R^1$ | $R^2$ | $R^2$ | $R^2$ |
|---|---|---|---|---|---|---|---|
| 9 | 20 EO | 3 | $C_{11}/C_{17}$ | H | Sulfo-succinat | H | H |
| 10 | 20 EO | 3 | $C_{11}/C_{17}$ | $C_{11}/C_{17}$ * | Sulfo-succinat | H | H |
| 11 | 5 EO | 2 | $C_{11}/C_{17}$ | H | Sulfo-succinat | Sulfo-succinat | H |
| 12 | 10 EO | 2 | $C_{11}/C_{17}$ | H | Sulfo-succinat | H | H |

* Alkylrest oder Alkylenrest der entsprechenden Säure

B) Anwendungstechnische Überprüfung

Die nachstehenden Formulierungen können durch einfaches Einrühren der Rezepturbestandteile in Wasser hergestellt werden.

Alle Rezepturen werden in Gewichtsprozent auf Feststoff berechnet angegeben.

Erklärung der eingesetzten Tenside nach CTFA-Namen:

1 = Disodium PEG-4 Cocoamido MIPA-Sulfosuccinate
2 = Disodiumlauroamphodiacetate (and) Sodium Lauryl Sulfate (and) Hexylene Glycol
3 = Cocoamidopropyl Hydroxysultaine
4 = Ricinoleamidopropyltrimonium Methosulfate
5 = PEG-200 Glyceryl Tallowate mod.
6 = Disodium Laureth Sulfosuccinate
7 = Sodium Laureth Sulfate
8 = Cocoamidopropyl Betaine
9 = PEG-200 Glyceryl Tallowate
10 = Disodiumcocoamphodiacetate
11 = PEG-80 Glyceryl Tallowate
12 = Cocoamidopropyl Betaine
13 = Glyceryl Stearate
14 = Laureth-6

Prüfmethoden:

Härteverträglichkeit: DIN 53 905

Hautverträglichkeit (Zein-Test):

Götte, Ernst, Chem. Phys. Appl. Surface Active Subst. Proc. Int. Congr.4 (1964) 83-90:
<200 mgN/100 ml = nicht irritierend
200-400 mgN/100 ml = leicht irritierend
>400 mgN/100 ml = irritierend

Oberflächenspannung:

Dr. R. Hensch; Fette, Seifen, Anstrichmittel, 72 (1970), S. 969-977

Viskosität:

Brookfield Rotationsviskositmeter (Becher und Spindel) gemessen bei 20 °C nach den Angaben des Geräteherstellers.

Bestimmung des Schaumvermögens nach Ross Miles in Anlehnung an DIN 53902 Teil 2 mit der Abänderung, daß folgende Maße und Mengen verwendet wurden:

| | |
|---|---|
| Innendurchmesser der Auslaufdüse: | 3,5 mm |
| Fallhöhe der Probelösung: | 940 mm |
| Menge der vorgelegten Probelösung: | 50 ml |
| Menge der zulaufenden Probelösung: | 200 ml |
| Meßtemperatur: | 40 °C |
| Lösungsmittel: | demineralisiertes Wasser |

Grundrezeptur: Hautreinigungsmittel und Make-up-Entferner für empfindliche Haut

| | | |
|---|---|---|
| Erfindungsgemäße Verbindung | 2 - 8 | Gew.-Teile |
| REWOTERIC[R*] AM B 14 [12]) | 2 - 8 | " |
| Hydroxyethylcellulose | 0,2 - 1,5 | " |
| demin. Wasser | ad 100 | |

* = Warenzeichen der Fa. REWO Chemie Werke GmbH, Steinau an der Straße

Grundrezeptur: Hautcreme

| | | |
|---|---|---|
| Erfindungsgemäße Verbindung | 1 - 5 | Gew.-Teile |
| Glycerinmonostearat | 2 - 10 | " |
| Cetylalkohol | 1 - 4 | " |
| Paraffinöl 3,5 ° E | 4 - 12 | " |
| Glycerin | 1 - 5 | " |
| demin. Wasser | ad 100 | |
| Konservierungsmittel | n. B. | |

Grundrezeptur: Spülmittel

| | | |
|---|---|---|
| Erfindungsgemäße Verbindung | 1 - 5 | Gew.-Teile |
| REWOPOL[R] *NL 3-28 [7)] | 1 - 30 | " |
| REWOTERIC[R] AM CAS [3)] | 1 - 5 | " |
| REWOPAL LA 6 [14)] | 1 - 10 | " |
| demin. Wasser | ad 100 | |

* = Warenzeichen der Fa. REWO Chemie Werke GmbH, Steinau an der Straße

Grundrezeptur: Duschbad

| | | |
|---|---|---|
| Erfindungsgemäße Verbindung | 2 - 8 | Gew.-Teile |
| REWOTERIC[R] AM G 30 [2)] | 5 - 15 | " |
| REWOTERIC[R] AM CAS [3)] | 2 - 6 | " |
| REWOQUAT[R*] RTM 50 [4)] | 1 - 3 | " |
| REWODERM[R*] LI S 75 [5)] | 1 - 4 | " |
| demin. Wasser | ad 100 | |

* = Warenzeichen der Fa. REWO Chemie Werke GmbH, Steinau an der Straße

Grundrezeptur: Haarshampoo

| | | |
|---|---|---|
| Erfindungsgemäße Verbindung | 2 - 8 | Gew. - Teile |
| REWOPOL$^R$ NL 3 - 28 [7)] | 4 - 10 | " |
| REWOTERIC$^R$ AM B 13 [8)] | 2 - 8 | " |
| REWODERM$^R$ LI 420 - 70 [9)] | 1 - 4 | " |
| demin. Wasser | ad 100 | |

Grundrezeptur: Babyshampoo

| | | |
|---|---|---|
| Erfindungsgemäße Verbindung | 2 - 8 | Gew. - Teile |
| REWOTERIC$^R$ AM 2 C NM [10)] | 2 - 8 | " |
| REWOTERIC$^R$ AM B 13 [8)] | 4 - 12 | " |
| REWOPOL$^R$ NL 3 - 28 [7)] | 2 - 10 | " |
| REWODERM$^R$ LI 48 - 50 [11)] | 1 - 4 | " |
| demin. Wasser | ad 100 | |

Tabelle 2

| Beispiel aus Tabelle 1 | Schaumvermögen [mm] | | Calciumhärteverträglichkeit | | Zeintest | Oberflächenspannung Lecomte du Noüy (Ringmethode) [mN/m] | |
|---|---|---|---|---|---|---|---|
| | sofort | nach 5 Min. | Punkte | Einstufung | [mgN/100ml] | 0,1 % | 0,01 % |
| Beispiel 4 | 145 | 135 | 75 | V | 26 | 37,4 | 41,3 |
| Beispiel 5 | 110 | 90 | 75 | V | - | 41,9 | 43,4 |
| Beispiel 6 | 125 | 115 | 75 | V | 20 | 42,0 | 43,9 |
| Beispiel 7 | 110 | 100 | 75 | V | 32 | 40,7 | 47,4 |
| Beispiel 9 | 125 | 110 | 75 | V | 18 | 41,3 | 44,8 |
| Beispiel 10 | 100 | 60 | 75 | V | 28 | 44,9 | 49,9 |
| Beispiel 11 | 120 | 110 | 75 | V | - | 35,7 | 43,9 |
| Beispiel 12 | 125 | 110 | 75 | V | 23 | 38,8 | 41,1 |

Tabelle 2 (Fortsetzung)

| Beispiel aus Tabelle 1 | Schaumvermögen [mm] sofort | Schaumvermögen [mm] nach 5 Min. | Calciumhärteverträglichkeit Punkte | Calciumhärteverträglichkeit Einstufung | Zeintest [mgN/100ml] | Oberflächenspannung Lecomte du Noüy (Ringmethode) [mN/m] 0,1 % | Oberflächenspannung Lecomte du Noüy (Ringmethode) [mN/m] 0,01 % |
|---|---|---|---|---|---|---|---|
| Vergleichsbeispiel REWOPOL® NL 3-28[1] | 200 | 180 | 75 | V | 300 | 34,2 | 32,9 |
| Vergleichsbeispiel REWOPOL® SB FA 30[2] | 170 | 160 | 75 | V | 250 | 28,0 | 31,4 |

CTFA-Namen

[1] = lfd. Nr. 7

[2] = lfd. Nr. 6

Testrezeptur: Duschbad

Die erfindungsgemäßen Beispiele sind in der Testrezeptur von einem Testpanel von 20 Personen (weiblich und männlich) bewertet worden bezüglich

- Anschäumvermögen
- Hautgefühl
- Hautgefühl der abgetrockneten Haut

Hautverträglichkeit: Reduzierung der Zeinwerte in Rezeptur Tabelle 4.

Tabelle 3

| Rezeptur | 1 | 2 | 3 |
|---|---|---|---|
| Beispiel 4 | – | 4 | – |
| Beispiel 10 | – | – | 4 |
| REWOPOL$^R$ SB Z [1)] | 4 | – | – |
| REWOTERIC$^R$ AM G 30 [2)] | 10,5 | 10,5 | 10,5 |
| REWOTERIC$^R$ AM CAS [3)] | 3 | 3 | 3 |
| REWOQUAT$^R$ RTM 50 [4)] | 1 | 1 | 1 |
| REWODERM$^R$ LI S 75 [5)] | 2,3 | 2,3 | 2,3 |
| demin. Wasser | ad 100 | ad 100 | ad 100 |
| pH – Wert, mit Citronensäure eingestellt | 6,5 | 6,5 | 6,5 |
| Viskosität mit NaCL eingestellt [mPas] | ca. 1000 | ca. 1000 | ca. 1000 |
| Schaumhöhe [mm] | 195/185 | 195/180 | 190/185 |
| Anschäumvermögen [A)] | gut | gut | gut |
| Schaumstruktur [A)] | feinblasig | cremig, feinblasig | cremig, feinblasig |
| Hautgefühl, nasse Haut [A)] | relativ glatt | glatt | glatt |
| Hautgefühl, trockene Haut [A)] | relativ glatt | glatt | glatt |

[A)] = Die Bewertung erfolgte nach einem abgestuften Bewertungssystem, wobei die oben angeführte Beurteilung den arithmetischen Mittelwert wiedergibt

Anschäumvermögen:

Bestimmung der Menge an gebildetem Schaum beim Verreiben zwischen den feuchten Händen (Händewaschen)

Tabelle 4

Testrezeptur: Hautreinigungspräparat, Reduzierung Zein-Werte

| | 1 | 2 | 3 |
|---|---|---|---|
| Beispiel 6 | 4,5 | - | - |
| REWOPOL® SB FA 30 [6] | - | 4,5 | - |
| REWOPOL® NL 3-28 [7] | 10,5 | 10,5 | 15 |
| demin. Wasser | 85 | 85 | 85 |
| pH-Wert, mit Citronensäure eingestellt | 6,5 | 6,5 | 6,5 |
| Bewertung der Schaumqualität | cremig, feinblasig | feinblasig | grobblasig |
| Hautgefühl nach dem Waschen auf der trockenen Haut | angenehm glatt | weich | etwas rauh |
| Zein-Werte Hautverträglichkeit | 104 mgN/ 100 ml | 270 mgN/ 100 ml | 300 mgN/ 100 ml |

Testrezeptur: Haarshampoo

Die erfindungsgemäßen Beispiele sind in der Rezeptur von 10 in der Anwendung erfahrenen Testpersonen auf

- Kämmbarkeit
- Volumen des Haares und Sitz der Frisur bewertet worden.

Tabelle 5

| Rezeptur | 1 | 2 |
|---|---|---|
| Beispiel 6<br>REWOPOL$^{R}$ NL 3-28 [7)]<br>REWOTERIC$^{R}$ AM B 13 [8)]<br>REWODERM$^{R}$ LI 420-70 [9)]<br>demin. Wasser | –<br>8<br>5<br>2,1<br>ad 100 | 3<br>6<br>4<br>2,1<br>ad 100 |
| pH-Wert, mit Citronensäure eingestellt<br>Viskosität mit Natriumchlorid eingestellt [mPas] | 6,5<br>ca. 1000 | 6,5<br>ca. 1000 |
| Kämmbarkeit | 3 | 5-6 |
| Volumen des Haares Sitz der Frisur | 3-4 | 6 |
| Schaumvermögen [mm] | 195/185 | 190/185 |

Bewertung der Kämmbarkeit:

1 - 3: schlecht durchzukämmen, das Haar setzt dem Kämmvorgang erheblichen Widerstand entgegen
4 - 7: das Haar läßt sich relativ gut durchkämmen, Kämmwiderstand verringert, je nach Haartyp ausreichend für ein Conditioning-Shampoo
8 - 10: vorbehalten für die nachfolgende Nachbehandlung durch sogenannte Hair-Rinse-Mittel

Bewertung des Haarvolumens und des Sitzes der Frisur

1 - 3: das Haar ist trocken oder strohig, schlechter Sitz der Frisur
4 - 7: das Haar ist weich und füllig, bei gleichzeitig gutem Sitz der Frisur
8 - 10: das Haar ist weich und glatt, jedoch zu locker, dadurch kein guter Sitz der Frisur.

Die Bewertung erfolgte nach einem abgestuften Punktesystem, wobei die oben angegebene Beurteilung den arithmetischen Mittelwert wiedergibt.

Testrezeptur: Babyshampoo bzw. Haar- und Körpershampoo für empfindliche Haut

Tabelle 6

| Rezeptur | 1 | 2 | 3 |
|---|---|---|---|
| Beispiel 6<br>Beispiel 10<br>REWOTERIC$^{R}$ AM 2 C NM [10)]<br>REWOTERIC$^{R)}$ AM B 13 [8)]<br>REWOPOL$^{R)}$ NL 3-28 [7)]<br>REWODERM$^{R)}$ LI 48-50 [11)]<br>demin. Wasser | –<br>–<br>3,5<br>7<br>7,5<br>3<br>ad 100 | 3<br>–<br>3,5<br>7<br>4,5<br>3<br>ad 100 | –<br>3<br>3,5<br>7<br>4,5<br>3<br>ad 100 |
| pH-Wert, mit Citronensäure eingestellt | 6,5 | 6,5 | 6,5 |
| Schaumvermögen [mm] | 195/185 | 195/185 | 195/185 |
| Zeinwerte [mgN/100 ml] (Hautverträglichkeit) | ~70 | ~30 | ~30 |
| Schaumstruktur | grobblasig | feinblasig | feinblasig |
| Hautgefühl, nasse und trockene Haut | etwas rauh | glatt weich | glatt weich |

Testrezeptur: Hautreinigungsmittel und Make-up-Entferner für empfindliche Haut

Tabelle 7

| Rezeptur | 1 | 2 |
|---|---|---|
| Beispiel 6<br>REWOPOL$^R$ SB FA 30 [6)]<br>REWOTERIC AM B 14 [12)]<br>Hydroxyethylcellulose<br>demin. Wasser | -<br>4<br>4,5<br>0,5<br>ad 100 | 4<br>-<br>4,5<br>0,5<br>ad 100 |
| pH-Wert, mit Citronensäure eingestellt | 6,5 | 6,5 |
| Zein-Werte [mgN/100 ml] (Hautverträglichkeit) | ca. 170 | <50 |

Der Einsatz der erfindungsgemäßen Sulfosuccinate in der vorliegenden Rezeptur ergibt ein sehr gut hautverträgliches Reinigungsmittel, was sich im niedrigen Zein-Wert zeigt im Vergleich zum Standard Sulfosuccinate REWOPOL$^R$ SB FA 30.

Tabelle 8 Testrezeptur: O/W-Hautcreme

| Rezeptur | 1 | 2 | 3 | 4 |
|---|---|---|---|---|
| Beispiel 4 | – | – | 2 | – |
| Beispiel 9 | – | – | – | 2 |
| Stearylalkohol-ethoxylat (3EO) | 2 | – | – | – |
| REWOMUL$^{R}$ MG [13] | 6 | 6 | 6 | 6 |
| REWOPOL$^{R}$ SB FA 30 [6] | – | 2 | – | – |
| Cetylalkohol | 2 | 2 | 2 | 2 |
| Paraffinöl 3,5°E | 8 | 8 | 8 | 8 |
| Glycerin | 3 | 3 | 3 | 3 |
| Konservierungsmittel | n.B. | n.B. | n.B. | n.B. |
| demin. Wasser | ad 100 | ad 100 | ad 100 | ad 100 |

Tabelle 8 Testrezeptur: O/W-Hautcreme (Fortsetzung)

| Rezeptur | 1 | 2 | 3 | 4 |
|---|---|---|---|---|
| Stabilität der Emulsion bei 5 °C, 20 °C und 40 °C | stabil | instabil | stabil | stabil |
| Aussehen | glatte, weiße Creme | griesig, Emulsion bricht | glatte,weiße Creme | glatte weiße Creme |

Bewertung der Eigenschaften auf der Haut durch 10 Testpersonen; mehr als 80 % beurteilten die Testrezepturen 3 und 4 als

- gut auf der Haut zu verstreichen
- gut in die Haut einziehend
- die Haut wird glatt und weich, ohne klebrig, fettig zu sein.

Die Vergleichsrezeptur 1 wird gleich bewertet, die Rezeptur 2 ist instabil.

Tabelle 9 Testrezeptur: Hautfreundliche Geschirrspülmittel zur manuellen Anwendung

| Rezeptur | 1 | 2 | 3 | 4 |
|---|---|---|---|---|
| Beispiel 6 | - | 3 | 1,5 | - |
| REWOPOL® NL 3-28 [7] | 18 | 18 | 18 | 18 |
| REWOTERIC® AM CAS [3] | 1,5 | - | 1,5 | - |
| REWOPAL LA 6 [4] | 1,5 | - | 1,5 | 3 |
| demin. Wasser | ad 100 | ad 100 | ad 100 | ad 100 |
| Viskosität [mPa.s] mit NaCl eingestellt auf ca. | 500 | 500 | 500 | 500 |
| Schaumvermögen [mm] | 195/185 | 195/180 | 195/185 | 190/180 |
| + 0,5 ml Olivenöl | 190/180 | 185/180 | 190/180 | 190/180 |
| + 1,0 ml Olivenöl | 190/185 | 180/175 | 190/180 | 190/180 |
| Zein-Werte (Hautverträglichkeit) [mgN/100 ml] | 220 | 220 | 190 | 250 |

**Patentansprüche**

**Patentansprüche für folgende Vertragsstaaten : BE, DK, DE, GB, FR, NL, IT, PT**

**1.** Fettsäurepolyolpolyethersulfosuccinate der allgemeinen Formel (I)

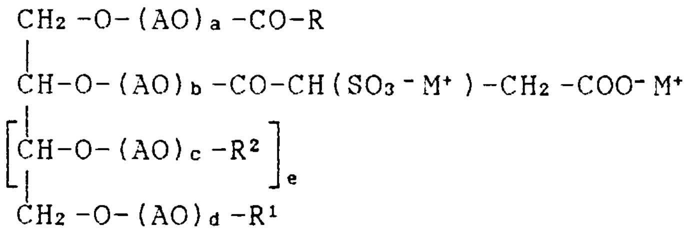

I

worin

R = ein geradkettiger oder verzweigter Alkyl- oder Alkylenrest mit 7 - 21 C-Atomen,
$R^1$ = H oder -CO-R,
$R^2$ = unabhängig voneinander H oder der Rest $-CO-CH(SO_3^-M^+)-CH_2-COO^-M^+$ sein kann,
A = gleich oder verschiedene Alkylenreste mit 2 - 3 C-Atomen sind,
a,b,c,d = gleich oder verschieden 0 - 5 sein können, wobei a + b + c • e + d = 2 - 25,
e = 1 - 3,
$M^+$ = Alkalimetallkation, Ammoniumion bedeuten.

**2.** Verbindungen der allgemeinen Formel (I), dadurch gekennzeichnet, daß

R = ein geradkettiger oder verzweigter Alkyl- oder Alkylenrest mit 11 - 17 C-Atomen ist,
$R^1$ = H oder -CO-R ist,
$R^2$ = unabhängig voneinander H oder der Rest $-CO-CH(SO_2-Na)-CH_2-COO-Na$ sein kann,
A = der Ethylenoxid- und/oder der Propylenoxidrest ist,
a,b,c,d = gleich oder verschieden 1 - 3 sein können, wobei a+b+c•e+d = 5 - 20 sind,
e = 3 und
$M^+$ = $Na^+$ ist.

**3.** Verbindungen der allgemeinen Formel (I) gemäß Anspruch 2, dadurch gekennzeichnet, daß a+b+c•e+d = 5 - 10, $R^1$, $R^2$ = Reste aus der natürlichen Mischung der Kokosfettsäure, Ölsäure, Ricinolsäure und/oder Laurinsäure sind.

**4.** Verfahren zur Herstellung von Verbindungen der allgemeinen Formel (I)

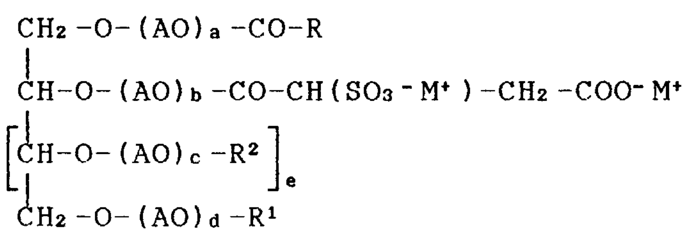

I

worin

R = ein geradkettiger oder verzweigter Alkyl- oder Alkylenrest mit 7 - 21 C-Atomen,
$R^1$ = H oder -CO-R,
$R^2$ = unabhängig voneinander H oder der Rest $-CO-CH(SO_3-M^+)-CH_2-COO-M^+$ sein kann,
A = gleich oder verschiedene Alkylenreste mit 2 - 3 C-Atomen sind,
a,b,c,d = gleich oder verschieden 0 - 5 sein können, wobei a + b + c • e + d = 2 - 25,
e = 1 - 3,
$M^+$ = Alkalimetallkation, Ammoniumion bedeuten,

dadurch gekennzeichnet, daß in erster Stufe Polyolverbindungen der allgemeinen Formel (II)

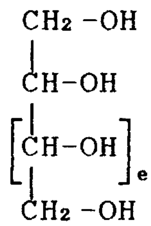

II

mit Alkylenoxiden in Gegenwart eines basischen Katalysators bei Temperaturen von 120 – 180 °C unter einem Druck von maximal 4 bar zu Verbindungen der allgemeinen Formel (III)

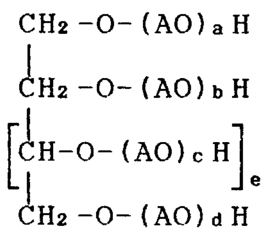

III

umgesetzt werden, wobei das Molverhältnis von Polyol zu Alkylenoxid zwischen 5 und 20 beträgt, und weitere Umsetzung mit Fettsäuren oder Fettsäureestern in Gegenwart eines Katalysators bei Temperaturen zwischen 120 und 180 °C zu Verbindungen der allgemeinen Formel (IV)

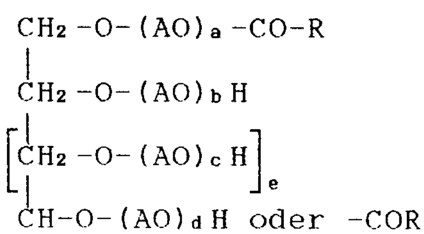

IV

und weitere Umsetzung dieser Verbindungen mit Maleinsäureanhydrid bei Temperaturen zwischen 60 und 80 °C zu den Halbestern und anschließende Sulfierung mit wässriger Natriumsulfit-Lösung zu den Verbindungen der allgemeinen Formel (V)

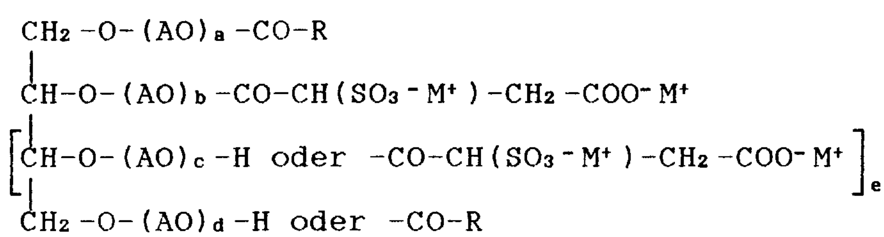

V

**5.** Verbindungen der allgemeinen Formel (I) gemäß Anspruch 4, dadurch gekennzeichnet, daß als Alkylenoxid Ethylenoxid und/oder Propylenoxid in Mengen von 5 – 20 Mol pro Mol Polyol und daß als Fettsäure (-ester) geradkettige gesättigte und/oder ungesättigte Verbindungen mit 11 – 17 C-Atomen im Kohlenwasserstoffrest in einem Molverhältnis von 1 – 2 : 1 zu alkoxyliertem Polyol und daß das Maleinsäureanhydrid in einem Molverhältnis von 1 – 3 : 1 zum Polyolalkoxylatester eingesetzt wird.

**6.** Wässrige Haarreinigungsmittel, enthaltend
a) 1 – 10 Gew. – Teile mindestens eine der Verbindungen der allgemeinen Formel (I) und
b) 1 – 20 Gew. – Teile mindestens eines Tensides aus der Gruppe der nichtionischen, amphoteren, zwitterionischen, ionischen Tenside und gegebenenfalls
c) 0,1 – 10 Gew. – Teile Verdickungsmittel, Duftstoffe, Konservierungsstoffe, Farbstoffe, Pflanzen – extrakte und sonstige Zusatz – und Hilfsstoffe und
d) ad 100 Wasser.

**7.** Wässriges Duschshampoo, enthaltend
a) 1 – 10 Gew. – Teile der Verbindungen der allgemeinen Formel (I)
b) 1 – 20 Gew. – Teile mindestens eines nichtionischen, amphoteren, zwitterionischen, ionischen Tensids und
c) 0,1 – 10 Gew. – Teile Verdickungsmittel, Duftstoffe, Konservierungsmittel, Farbstoffe, Pflanzen – extrakte und sonstige Zusatz – und Hilfsstoffe und gegebenenfalls
d) ad 100 Wasser.

**8.** Wässriges Spülmittel, enthaltend
a) 1 – 10 Gew. – Teile mindestens einer der Verbindungen der allgemeinen Formel (I) und
b) 1 – 30 Gew. – Teile mindestens eines Tensids aus der Gruppe der nichtionischen, amphoteren, zwitterionischen, ionischen Tenside und gegebenenfalls
c) 0,1 – 10 Gew. – Teile Verdickungsmittel, Duftstoffe, Konservierungsmittel, Farbstoffe, Pflanzen – extrakte und sonstige Zusatz – und Hilfsstoffe und gegebenenfalls
d) ad 100 Wasser.

**9.** Hautcreme, enthaltend
a) 1 – 10 Gew. – Teile mindestens einer der Verbindungen der allgemeinen Formel (I) und
b) 1 – 10 Gew. – Teile mindestens eines Tensids aus der Gruppe der nichtionischen, amphoteren, zwitterionischen, ionischen Tenside und gegebenenfalls
c) 2 – 10 Gew. – Teile pflanzliche oder mineralische Oele, Esteroele
d) 1 – 5 Gew. – Teile Konsistenzgeber
e) 0,5 – 5,0 Gew. – Teile Duftstoffe, Farbstoffe, Konservierungsmittel
f) ad 100 Wasser.

**10.** Babyshampoo, enthaltend
a) 1 – 10 Gew. – Teile mindestens einer der Verbindungen der allgemeinen Formel (I) und
b) 0,1 – 20 Gew. – Teile mindestens eines Tensids aus der Gruppe der nichtionischen, amphoteren, zwitterionischen, ionischen Tenside und gegebenenfalls
c) 0,1 – 10 Gew. – Teile Verdickungsmittel, Duftstoffe, Konservierungsmittel, Farbstoffe, Pflanzen – extrakte und sonstige Zusatz – und Hilfsstoffe und gegebenenfalls
d) ad 100 Wasser.

**Patentansprüche für folgenden Vertragsstaat : ES**

**1.** Verfahren zur Herstellung von Verbindungen der allgemeinen Formel (I)

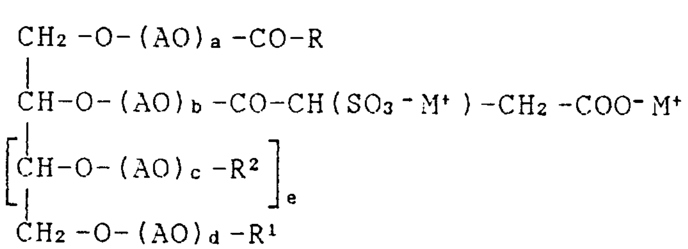

I

worin
R = ein geradkettiger oder verzweigter Alkyl – oder Alkylenrest mit 7 – 21 C – Atomen,
$R^1$ = H oder – CO – R,

$R^2$ = unabhängig voneinander H oder der Rest $-CO-CH(SO_3^-M^+)-CH_2-COO^-M^+$ sein kann,
A = gleich oder verschiedene Alkylenreste mit 2 – 3 C – Atomen sind,
a,b,c,d = gleich oder verschieden 0 – 5 sein können, wobei a + b + c • e + d = 2 – 25,
e = 1 – 3,
$M^+$ = Alkalimetallkation, Ammoniumion bedeuten,

dadurch gekennzeichnet, daß in erster Stufe Polyolverbindungen der allgemeinen Formel (II)

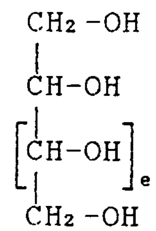

II

mit Alkylenoxiden in Gegenwart eines basischen Katalysators bei Temperaturen von 120 – 180 °C unter einem Druck von maximal 4 bar zu Verbindungen der allgemeinen Formel (III)

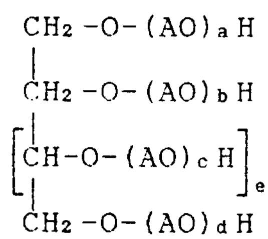

III

umgesetzt werden, wobei das Molverhältnis von Polyol zu Alkylenoxid zwischen 5 und 20 beträgt, und weitere Umsetzung mit Fettsäuren oder Fettsäureestern in Gegenwart eines Katalysators bei Temperaturen zwischen 120 und 180 °C zu Verbindungen der allgemeinen Formel (IV)

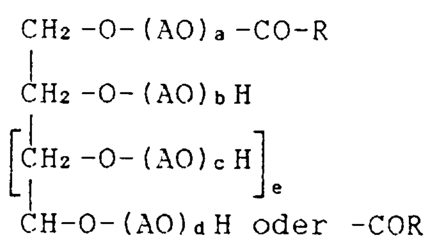

IV

und weitere Umsetzung dieser Verbindungen mit Maleinsäureanhydrid bei Temperaturen zwischen 60 und 80 °C zu den Halbestern und anschließende Sulfierung mit wässriger Natriumsulfit – Lösung zu den Verbindungen der allgemeinen Formel (V)

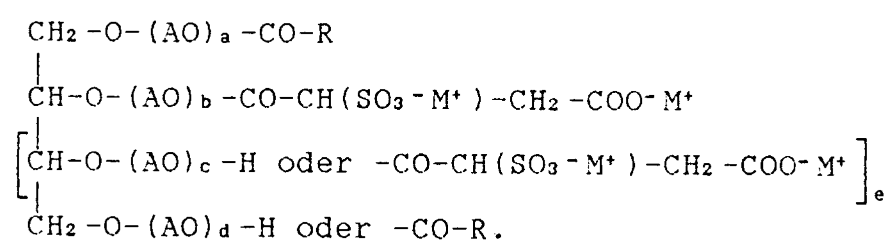

V

2. Verfahren zur Herstellung der Verbindungen der allgemeinen Formel (I), dadurch gekennzeichnet, daß

| | |
|---|---|
| R = | ein geradkettiger oder verzweigter Alkyl- oder Alkylenrest mit 11 - 17 C-Atomen ist, |
| $R^1$ = | H oder -CO-R ist, |
| $R^2$ = | unabhängig voneinander H oder der Rest $-CO-CH(SO_2^-Na)-CH_2-COO^-Na$ sein kann, |
| A = | der Ethylenoxid- und/oder der Propylenoxidrest ist, |
| a,b,c,d = | gleich oder verschieden 1 - 3 sein können, wobei a+b+c•e+d = 5 - 20 sind, |
| e = | 3 und |
| $M^+$ = | $Na^+$ ist. |

3. Verfahren zur Herstellung der Verbindungen der allgemeinen Formel (I) gemäß Anspruch 2, dadurch gekennzeichnet, daß a+b+c•e+d = 5 - 10, $R^1$, $R^2$ = Reste aus der natürlichen Mischung der Kokosfettsäure, Ölsäure, Ricinolsäure und/oder Laurinsäure sind.

4. Verfahren zur Herstellung der Verbindungen der allgemeinen Formel (I) gemäß Anspruch 1, dadurch gekennzeichnet, daß als Alkylenoxid Ethylenoxid und/oder Propylenoxid in Mengen von 5 - 20 Mol pro Mol Polyol und daß als Fettsäure (-ester) geradkettige gesättigte und/oder ungesättigte Verbindungen mit 11 - 17 C-Atomen im Kohlenwasserstoffrest in einem Molverhältnis von 1 - 2 : 1 zu alkoxyliertem Polyol und daß das Maleinsäureanhydrid in einem Molverhältnis von 1 - 3 : 1 zum Polyolalkoxylatester eingesetzt wird.

5. Verwendung der gemäß den Ansprüchen 1 und 2 hergestellten Verbindungen zur Herstellung wässriger Haarreinigungsmittel, enthaltend
a) 1 - 10 Gew.-Teile mindestens eine der Verbindungen der allgemeinen Formel (I) und
b) 1- 20 Gew.-Teile mindestens eines Tensides aus der Gruppe der nichtionischen, amphoteren, zwitterionischen, ionischen Tenside und gegebenenfalls
c) 0,1 - 10 Gew.-Teile Verdickungsmittel, Duftstoffe, Konservierungsstoffe, Farbstoffe, Pflanzenextrakte und sonstige Zusatz- und Hilfsstoffe und
d) ad 100 Wasser.

6. Verwendung der gemäß den Ansprüchen 1 und 2 hergestellten Verbindungen zur Herstellung von wässrigem Duschshampoo, enthaltend
a) 1 - 10 Gew.-Teile der Verbindungen der allgemeinen Formel (I)
b) 1 - 20 Gew.-Teile mindestens eines nichtionischen, amphoteren, zwitterionischen, ionischen Tensids und
c) 0,1 - 10 Gew.-Teile Verdickungsmittel, Duftstoffe, Konservierungsmittel, Farbstoffe, Pflanzenextrakte und sonstige Zusatz- und Hilfsstoffe und gegebenenfalls
d) ad 100 Wasser.

7. Verwendung der gemäß den Ansprüchen 1 und 2 hergestellten Verbindungen zur Herstellung wässriger Spülmittel, enthaltend
a) 1 - 10 Gew.-Teile mindestens einer der Verbindungen der allgemeinen Formel (I) und
b) 1 - 30 Gew.-Teile mindestens eines Tensids aus der Gruppe der nichtionischen, amphoteren, zwitterionischen, ionischen Tenside und gegebenenfalls
c) 0,1 - 10 Gew.-Teile Verdickungsmittel, Duftstoffe, Konservierungsmittel, Farbstoffe, Pflanzenextrakte und sonstige Zusatz- und Hilfsstoffe und gegebenenfalls
d) ad 100 Wasser.

8. Verwendung der gemäß den Ansprüchen 1 und 2 hergestellten Verbindungen zur Herstellung von Hautcreme, enthaltend
a) 1 - 10 Gew.-Teile mindestens einer der Verbindungen der allgemeinen Formel (I) und
b) 1 - 10 Gew.-Teile mindestens eines Tensids aus der Gruppe der nichtionischen, amphoteren, zwitterionischen, ionischen Tenside und gegebenenfalls
c) 2 - 10 Gew.-Teile pflanzliche oder mineralische Oele, Esteroele
d) 1 - 5 Gew.-Teile Konsistenzgeber
e) 0,5 - 5,0 Gew.-Teile Duftstoffe, Farbstoffe, Konservierungsmittel
f) ad 100 Wasser.

**9.** Verwendung der gemäß den Ansprüchen 1 und 2 hergestellten Verbindungen zur Herstellung von Babyshampoo, enthaltend

a) 1 – 10 Gew. – Teile mindestens einer der Verbindungen der allgemeinen Formel (I) und

b) 0,1 – 20 Gew. – Teile mindestens eines Tensids aus der Gruppe der nichtionischen, amphoteren, zwitterionischen, ionischen Tenside und gegebenenfalls

c) 0,1 – 10 Gew. – Teile Verdickungsmittel, Duftstoffe, Konservierungsmittel, Farbstoffe, Pflanzen – extrakte und sonstige Zusatz – und Hilfsstoffe und gegebenenfalls

d) ad 100 Wasser.

Europäisches Patentamt

# EUROPÄISCHER RECHERCHENBERICHT

Nummer der Anmeldung

EP 92 11 1641

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| A | US-A-4 247 425 (R.R. EGAN ET AL.)<br>* das ganze Dokument *<br>--- | 1-3,5-7,10 | C07C309/17<br>C11D1/06<br>C11D1/12<br>C11D3/20 |
| A | US-A-4 256 611 (R.R. EGAN ET AL.)<br>* das ganze Dokument *<br>--- | 1-3,5-7,10 | |
| A | GB-A-1 054 244 (DUTTON AND REINISCH LIMITED)<br>* das ganze Dokument *<br>--- | 1-3,5-7,10 | |
| A | FR-A-2 025 943 (LA CITRIQUE BELGE N.V.)<br>* das ganze Dokument; insbesondere Seite 6, Zeile 20 - Zeile 22 *<br>----- | 1-3,5,8 | |

RECHERCHIERTE SACHGEBIETE (Int. Cl.5)

C07C

Der vorliegende Recherchenbericht wurde für alle Patentansprüche erstellt

| Recherchenort | Abschlußdatum der Recherche | Prüfer |
|---|---|---|
| DEN HAAG | 08 FEBRUAR 1993 | FINK D.G. |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPO FORM 1503 03.82 (P0403)